# FASCICULE DE BREVET EUROPEEN

(11) **EP 1 563 824 B1**
(45) Date de publication et mention de la délivrance du brevet: **18.06.2008**
(21) Numéro de dépôt: 05290249.1
(22) Date de dépôt: 11.02.2005
(51) Int. Cl.: A61K 8/92, A61Q 1/10

(54) **Composition de revêtement des fibres kératiniques comprenant une cire microcristalline collante et des fibres**
Beschichtungszusammensetzung für Keratinfasern enthaltend ein klebriges mikrokristallines Wachs and Fasern
Composition for coating keratin fibers comprising a tacky microscrystalline wax and fibers

(30) Priorité: 13.02.2004 US 543997 P
(43) Date de publication de la demande: 17.08.2005
(73) Titulaire: L'ORÉAL, 75008 Paris (FR)
(72) Inventeur: Feng, Sue, Edison, NJ 08820 (US)
(74) Mandataire: Boulard, Denis

(56) Documents cités:
- EP-A- 1 157 683
- EP-A- 1 201 221
- EP-A- 1 400 234
- EP-A- 1 424 058
- WO-A-01/03653
- FR-A- 2 629 713

## Description

La présente invention a pour objet une composition cosmétique de revêtement des fibres kératiniques comprenant une cire dite collante et des fibres. L'invention concerne aussi un procédé cosmétique de maquillage ou de traitement des fibres kératiniques telles que les cils, les sourcils, les cheveux.
L'invention a également pour objet une composition de soin ou de maquillage des yeux tel qu'un eye liner ou un fard à paupières.

La composition selon l'invention peut être une composition de maquillage des fibres kératiniques, une base de maquillage des fibres kératiniques ou base-coat, une composition à appliquer sur un maquillage, dite encore top-coat, ou bien encore une composition de traitement des fibres kératiniques dite encore basecoat. Plus spécialement, la composition selon l'invention est un mascara.

Par mascara, on entend une composition destinée à être appliquée sur les cils : il peut s'agir d'une composition de maquillage des cils, une base de maquillage des cils, une composition à appliquer sur un mascara, dite encore top-coat, ou bien encore une composition de traitement cosmétique des cils. Le mascara est plus particulièrement destiné aux cils d'êtres humains, mais également aux faux-cils.

Les mascaras sont couramment préparés selon deux types de formulation : les mascaras aqueux, dits mascaras crèmes, sous forme d'émulsion de cires dans l'eau ; les mascaras anhydres ou à faible teneur en eau, dits mascaras waterproofs, sous forme de dispersions de cires dans des solvants organiques.
Il est connu d'employer diverses cires pour la formulation des mascaras comme celles décrites dans le document WO-A-91/12793, par exemple la cire d'abeille, la cire de candellila, la cire de carnauba, ou bien encore la cire de polyéthylène.

Toutefois, lorsque les mascaras contiennent certaines cires en une teneur élevée (en particulier en une teneur supérieure à 10%) comme la cire de carnauba, la cire de son de riz
ou la cire de polyéthylène, le maquillage des cils obtenu présente un aspect granuleux conférant ainsi un maquillage non lisse et non homogène, défauts qui rendent le maquillage inesthétique.

Par ailleurs, pour obtenir un mascara présentant de bonnes propriétés chargeantes, c'est-à-dire obtenir un maquillage épais des cils, il est possible d'incorporer dans le mascara une ou plusieurs cires en une teneur totale supérieure à 25 % en poids du poids total du mascara. Or en utilisant les cires classiques comme la cire d'abeille, la cire de candellila, ou la cire de carnauba dans ces teneurs élevées, la composition de mascara devient très consistante, voire trop compacte, et ne peut pas être appliquée facilement sur les cils avec les applicateurs de brosse à mascara couramment utilisés. Le mascara trop épais est déposé sur les cils sous forme de paquets et le maquillage ainsi obtenu ne présente pas l'aspect lisse recherché ; le maquillage n'est pas homogène et a un aspect inesthétique.
En outre, certaines cires comme la cire d'orange, la cire de lanoline, employées à des teneurs supérieures à 25 % en poids conduisent à des compositions qui ne sont pas suffisamment stables, notamment après un stockage de deux semaines à température ambiante (25 °C) : la composition prend en masse (augmentation importante de la viscosité) ou présente un déphasage qui s'observe visuellement à l'oeil nu. La composition est alors inappropriée pour être appliquée sur les cils.

D'autre part, une autre propriété recherchée en mascara est l'allongements de cils. Pour obtenir un tel effet, il est connu d'utiliser en tant qu'additif des fibres.
Toutefois, les compositions cosmétiques de l'art antérieur contenant des fibres présentent un certain nombre d'inconvénients, tels qu'un maquillage hétérogène et peu chargeant (peu volumateur). En particulier, les compositions de mascara contenant des fibres de l'art antérieur ne permettent pas d'obtenir un effet d'allongement optimal des cils après l'application de la composition soit en raison d'une mauvaise orientation et une répartition aléatoire des fibres sur les cils (qui ne se situent pas dans le prolongement des cils), soit en raison d'une mauvaise accroche sur le cil car les fibres glissent sur le cil et au final peu de fibres et de produit sont déposés sur le support.
L'effet obtenu par les compositions de mascara contenant des fibres de l'art antérieur est donc souvent esthétiquement inacceptable, notamment dans le cas de cils fournis et/ou longs et/ou recourbés, pour lesquels on obtient un aspect des cils, dit « sapin de noël », particulièrement disgracieux avec un aspect non lisse des cils.
Un autre inconvénient majeur lié à l'utilisation de fibres dans les composition de l'art antérieur est la grande difficulté d'obtenir un effet volumateur (chargeant) car l'accroche du produit sur les cils est défavorisé par le glissement des fibres sur la surface du cil.

Le but de la présente invention est de proposer une composition de revêtement des fibres kératiniques permettant d'obtenir un maquillage des fibres kératiniques homogène et lisse, avec une bonne répartition des fibres le long du cil, et chargeant.
Les inventeurs ont découvert qu'une telle composition pouvait être obtenue en utilisant une cire particulière présentant des propriétés collantes (collant élevé) et des fibres. Cette cire permet d'obtenir une composition de revêtement des fibres kératiniques, en particulier un mascara qui s'applique facilement sur les cils, ayant une bonne accroche sur les cils et qui conduit à la formation d'un maquillage lisse et homogène avec un effet allongeant et chargeant (volumateur) des cils.
Il est connu d'une part, par exemple du document EP1400234, des mascaras comprenant des cires et des fibres. Il en est de même avec le document WO 0103653. On connaît également, par le document EP1424058, l'utilisation, dans des mascaras, de cires présentant des paramètres de dureté et de collant spécifiques. Toutefois l'association de fibres et de ces cires spécifiques, en vue d'obtenir l'effet revendiqué ci-dessus, n'est nullement décrite ni suggérée dans ce document.
De façon plus précise, l'invention a pour objet une composition de revêtement des fibres kératiniques comprenant, dans un milieu cosmétiquement acceptable, une cire microcristalline ayant un collant supérieur ou égal à 0,7 N.s et une dureté inférieure ou égale à 3,5 MPa et des fibres. Par "cire microcristalline", on entend un cire dérivée du pétrole et présentant des cristaux plus fins que les cristaux de la cire de paraffine.
L'invention a également pour objet un procédé cosmétique de maquillage ou de soin non thérapeutique des fibres kératiniques, en particulier des cils, comprenant l'application sur les fibres kératiniques d'une composition telle que définie précédemment.
L'invention a aussi pour objet l'utilisation d'une composition telle que définie précédemment pour obtenir un maquillage des fibres kératiniques, en particulier des cils, homogène et/ou lisse et un bon effet allongeant des fibres kératiniques maquillées et/ou un effet volumateur.
L'invention a encore pour objet l'utilisation de l'association d'une cire microcristalline ayant un collant supérieur ou égal à 0,7 N.s et une dureté inférieure ou égale à 3,5 MPa et de fibres, associée à des fibres dans une composition de maquillage des fibres kératiniques, pour obtenir un maquillage des fibres kératiniques homogène et/ou lisse et un effet allongeant et/ ou volumateur des fibres kératiniques maquillés.
On entend par « milieu cosmétiquement acceptable » un milieu cosmétique compatible avec les cils ou la peau.

### 1) Cire microcristalline

Par "cire", on entend au sens de la présente invention, un composé gras lipophile, solide à température ambiante (25°C) et pression atmosphérique (760 mm de Hg, soit 105 Pa), à changement d'état solide/liquide réversible, ayant une température de fusion supérieure à 30°C et mieux supérieure à 55 °C et pouvant aller jusqu'à 200° C, notamment jusqu'à 120 °C.

En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.
Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.
Le protocole de mesure est le suivant :
Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

La cire microcristalline dite collante, présente dans la composition selon l'invention a un collant supérieur ou égal à 0,7 N.s, notamment allant de 0,7 N.s à 30 N.s, de préférence supérieur ou égal à 0,8 N.s, notamment allant de 0,8 N.s à 10 N.s, et mieux allant de 0,8 N.s à 5 N.s.

La cire microcristalline de la composition selon l'invention a une dureté inférieure ou égale à 3,5 MPa, en particulier allant de 0,01 à 3,5 MPa, de préférence allant de 0,05 MPa à 3 MPa, plus préférentiellement allant de 0,1 MPa à 2 MPa, et mieux de 0, 2 à 1 MPa.

Le collant de la cire microcristalline est mesuré à 20 °C à l'aide du texturomètre vendu sous la dénomination TA-TX2i par la société RHEO, équipé d'un mobile en polymère acrylique en forme de cône formant un angle de 45°, en mesurant l'évolution de la force (force de compression ou force d'étirement) (F) en fonction du temps, pendant l'opération suivante :
Le mobile est déplacé à la vitesse de 0,5 mm /s puis pénètre dans la cire jusqu'à une profondeur de pénétration de 2 mm. Lorsque le mobile a pénétré dans la cire à la profondeur de 2 mm, le mobile est maintenu fixe pendant 1 seconde (correspondant au temps de relaxation) puis est retiré à la vitesse de 0,5 mm/s. Pendant le temps de relaxation, la force (force de compression) décroît fortement jusqu'à devenir nulle puis, lors du retrait du mobile, la force (force d'étirement) devient négative pour ensuite croître à nouveau vers la valeur 0. Le collant correspond à l'intégrale de la courbe de la force en fonction du temps pour la partie de la courbe correspondant aux valeurs négatives de la force (force d'étirement). La valeur du collant est exprimée en N.s.

Pour effectuer la mesure du collant de la cire, la cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure du collant.

La dureté est déterminée par la mesure de la force en compression mesurée à 20 °C à l'aide du texturomètre vendu sous la dénomination « TA-TX2i^{®} » par la société RHEO, équipé d'un mobile en inox en forme de cylindre d'un diamètre de 2 mm en mesurant l'évolution de la force (force de compression ou force d'étirement) (F) en fonction du temps, pendant l'opération suivante :
Le mobile est déplacé à la vitesse de 0,1 mm/s puis pénètre dans la cire jusqu'à une profondeur de pénétration de 0,3 mm. Lorsque le mobile a pénétré dans la cire à la profondeur de 0,3 mm, le mobile est maintenu fixe pendant 1 seconde (correspondant au temps de relaxation) puis est retiré à la vitesse de 0,1 mm/s. Pendant le temps de relaxation, la force (force de compression) décroît fortement jusqu'à devenir nulle puis lors du retrait du mobile, la force (force d'étirement) devient négative pour ensuite croître à nouveau vers la valeur 0. La dureté correspond à la force de compression maximale mesurée entre la surface du mobile et la cire au moment de leur mise en contact. La valeur de cette force est exprimée en MPa.
Pour effectuer la mesure de la dureté, la cire est fondue à une température égale au point de fusion de la cire + 20 °C. La cire fondue est coulée dans un récipient de 30 mm de diamètre et de 20 mm de profondeur. La cire est recristallisée à température ambiante (25°C) pendant 24 heures, puis la cire est conservée pendant au moins 1 heure à 20 °C avant d'effectuer la mesure de dureté.

Comme cire microcristallline collante, on peut utiliser la cire commercialisée sous la référence SP18 par la société STRAHL and PITSCH qui présente une dureté d'environ 0,46 MPa et une valeur de collant d'environ 1 N.s.

La cire microcristalline peut être présente dans la composition selon l'invention en une teneur allant de 0,5 % à 60 % en poids, par rapport au poids total de la composition, de préférence allant de 2 % à 40 % en poids, et plus préférentiellement allant de 5 % à 20 % en poids.

### 2) Fibres

Par "fibre", il faut comprendre un objet de longueur L et de diamètre D tel que L soit supérieur à D, et de préférence très supérieur à D, D étant le diamètre du cercle dans lequel s'inscrit la section de la fibre. En particulier, le rapport UD (ou facteur de forme) est choisi dans la gamme allant de 3,5 à 2500, de préférence de 5 à 500, et mieux de 5 à 150.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 µm à 10 mm, de préférence de 0,1 mm à 5 mm et mieux de 0,3 mm à 3 mm. Leur section peut être comprise dans un cercle de diamètre allant de 2 nm à 500 µm, de préférence allant de 100 nm à 100 µm et mieux de 1 µm à 50 µm. Le poids ou titre des fibres est souvent donné en denier ou décitex et représente le poids en gramme pour 9 km de fil. De préférence, les fibres selon l'invention ont un titre choisi dans la gamme allant de 0,01 à 10 à deniers, de préférence de 0,1 à 2 deniers et mieux de 0,3 à 0,7 deniers.

Les fibres peuvent être celles utilisées dans la fabrication des textiles et notamment des fibres de soie, de coton, de laine, de lin, des fibres de cellulose - notamment extraites notamment du bois, des légumes ou des algues -, de rayonne, de polyamide (Nylon^{®}), de viscose, d'acétate notamment d'acétate de rayonne, de polymère acrylique notamment de polyméthacrylate de méthyle ou de poly 2-hydroxyéthyl méthacrylate, de polyoléfine et notamment de polyéthylène ou de polypropylène, de verre, de silice, de carbone notamment sous forme graphite, de polytétrafluoroéthylène (comme le Téflon^{®}), de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/polyester.
De préférence, les fibres sont des fibres de polyamide ((Nylon^{®}).

On peut aussi utiliser les fibres utilisées en chirurgie comme les fibres synthétiques résorbables préparées à partir d'acide glycolique et caprolactone ("Monocryl" de chez Johnson & Johnson) ; les fibres synthétiques résorbables du type copolymère d'acide lactique et d'acide glycolique ("Vicryl" de chez Johnson & Johnson) ; les fibres de polyester téréphtalique ("Ethibond" de chez Johnson & Johnson) et les fils d'acier inoxydable ("Acier" de chez Johnson & Johnson).

Par ailleurs, les fibres peuvent être traités ou non en surface, enrobées ou non d'une couche de protection. Comme fibres enrobées utilisables dans l'invention, on peut citer des fibres de polyamides enrobées de sulfure de cuivre pour un effet anti-statique (par exemple le "R-STAT" de chez Rhodia) ou un autre polymère permettant une organisation particulière des fibres (traitement de surface spécifique). On peut encore citer les fibres enrobées par des pigments minéraux ou organiques, tels que les pigments cités plus loin dans la demande.

De préférence, on utilise des fibres d'origine synthétique et en particulier des fibres organiques, comme celles utilisées en chirurgie.

Les fibres utilisables dans la composition selon l'invention sont préférentiellement des fibres de polyamide, de cellulose ou de polyéthylène. Leur longueur (L) peut aller de 0,1 mm à 5 mm, de préférence de 0,25 mm à 1,6 mm et leur diamètre moyen peut aller de 1 µm à 50 µm. En particulier, on peut utiliser les fibres de polyamide commercialisées par les Etablissements P. Bonte sous le nom de "Polyamide 0.9 Dtex 3 mm", ayant un diamètre moyen de 6 µm, un titre d'environ 0.9 dtex et une longueur allant de 0,3 mm à 5 mm ou bien encore les fibres de polyamides vendues sous la dénomination Fiberlon 931-D1-S par la société LCW, ayant un titre d'environ 0,9 dtex et une longueur d'environ 0,3 mm. On peut aussi utiliser les fibres de celluloses (ou de rayonne) ayant un diamètre moyen de 50 µm et une longueur allant de 0,5 mm à 6 mm comme celles vendues sous le nom de "Natural rayon flock fiber RC1BE - N003 - M04" par la société Claremont Flock. On peut également utiliser des fibres de polyéthylène comme celles vendues sous le nom de "Shurt Stuff 13 099 F" par la société Mini Fibers.

On peut également utiliser les fibres élastomériques c'est à dire des fibres qui, lorsque soumises à une contrainte d'étirement (par exemple de 30% relativement à leur longueur initiale), reviennent à une longueur sensiblement identique à leur longueur initiale lorsque cesse la contrainte. Comme fibre élastomérique, on peut citer les fibres de polyuréthane telle que l'élastane (ou Spandex^{®}), les fibres comprenant au moins 85% en poids de polyuréthane segmenté telles que le Lycra^{®} commercialisées par Dupont de Nemours, l'elastodiène ou encore les fibres caoutchouteuses issues du caoutchouc naturel. Ces fibres élastomériques peuvent être vulcanisées ou non.

La composition selon l'invention peut également comprendre des fibres dites "rigides", par opposition aux fibres citées précédemment, qui ne sont pas des fibres rigides.
Les fibres rigides, initialement sensiblement droites, lorsqu'elles sont placées dans un milieu dispersant, ne voient pas leur forme sensiblement modifiée, ce qui se traduit par la condition angulaire définie ci-après, reflétant une forme que l'on peut qualifier de toujours, sensiblement droite, linéaire. Cette condition d'angle reflète la rigidité des fibres qui peut difficilement être exprimée par un autre paramètre pour des objets ayant une taille aussi faible que les fibres rigides.

La rigidité des fibres se traduit par la condition angulaire suivante : avantageusement, au moins 50 % en nombre, de préférence au moins 75 % en nombre, et mieux au moins 90 % en nombre des fibres sont telles que l'angle formé entre la tangente à l'axe central longitudinal de la fibre et la droite reliant ladite extrémité au point sur l'axe central longitudinal de la fibre correspondant à la moitié de la longueur de la fibre soit inférieur à 15 ° et l'angle formé entre la tangente à l'axe central longitudinal de la fibre en un point situé à mi-longueur de la fibre et la droite reliant l'une des extrémités au point sur l'axe central longitudinal de la fibre correspondant à la moitié de la longueur de fibre soit inférieur ou égal à 15 °, pour une même longueur de fibre allant de 0,8 mm à 5 mm, de préférence allant de 1 mm à 4 mm, de préférence allant de 1 mm à 3 mm, et mieux de 2 mm.
Avantageusement, l'angle, mentionné ci-dessus, est mesuré aux deux extrémités de la fibre et en un point situé à mi-longueur de la fibre, en d'autres termes, on effectue dans ce cas trois mesures et la moyenne des angles mesurés est inférieure ou égale à 15°.
Notamment, la tangente, en tout point de la fibre, forme un angle inférieur à 15°.
Dans la présente demande, l'angle formé par la tangente en un point de la fibre est l'angle formé entre la tangente à l'axe central longitudinal de la fibre audit point de la fibre et la droite reliant l'extrémité de la fibre la plus proche dudit point au point sur l'axe central longitudinal de la fibre correspondant à la moitié de la longueur de la fibre.

Généralement, les fibres rigides utilisables dans la composition selon l'invention ont la même longueur de fibre ou une longueur sensiblement identique.

Plus précisément lorsque l'on observe au microscope, avec un objectif permettant un grossissement de 2,5 et avec une vision plein champ, un milieu dans lequel sont dispersées les fibres rigides à une concentration des fibres de 1 % en poids, un nombre majoritaire de fibres rigides, c'est-à-dire au moins 50 % en nombre des fibres rigides, de préférence au moins 75 % en nombre des fibres rigides, et mieux au moins 90 % en nombre des fibres rigides, doivent satisfaire à la condition angulaire définie plus haut. La mesure conduisant à la valeur de l'angle est effectuée pour une même longueur de fibres, cette longueur est comprise dans la gamme allant de 0,8 mm à 5 mm, de préférence de 1 à 4 mm, de préférence de 1 à 3 mm, et mieux de 2 mm.
Le milieu dans lequel est réalisée l'observation est un milieu dispersant assurant une bonne dispersion des fibres rigides, par exemple de l'eau, un gel aqueux d'argile ou de polyuréthane associatif. On peut même réaliser une observation directe de la composition contenant les fibres rigides. Un échantillon de la composition ou de la dispersion préparée est placée entre lame et lamelle pour l'observation au microscope avec un objectif permettant un grossissement de 2,5 et avec une vision plein champ. La vision plein champ permet de voir les fibres dans leur intégralité.

Les fibres rigides peuvent être choisies parmi les fibres d'un polymère synthétique choisi parmi les polyesters, les polyuréthanes, les polymères acryliques, les polyoléfines, les polyamides, en particulier les polyamides non aromatiques et les polyimides-amides aromatiques.

Comme exemples de fibres rigides, on peut citer les fibres :
- de polyesters, telles que celles obtenues par découpe de fils vendus sous les dénominations FIBRE 255-100-R11-242T TAILLE 3 MM (section octalobée), FIBRE 265-34-R11-56T TAILLE 3 MM (section ronde), FIBRE COOLMAX 50-34-591 TAILLE 3 MM (section tétralobée) par la société DUPONT DE NEMOURS ;
- de polyamide, telles que celles vendues sous les dénominations TRILOBAL NYLON 0.120-1.8 DPF ; TRILOBAL NYLON 0.120-18 DPF ; NYLON 0.120-6 DPF par la société Cellusuede products ; ou obtenues par découpe de fils vendus sous la dénomination FIBRE NOMEX BRAND 430 TAILLE 3 MM par la société DUPONT DE NEMOURS ;
- de polyimide-amide telles que celles vendues sous les dénomination "KERMEL", " KERMEL TECH" par la société RHODIA ;
- de poly-(p-phénylène-téréphtalamide) (ou d'aramide) notamment vendues sous la dénomination Kevlar^{®} par la société DUPONT DE NEMOURS ;
- des fibres à structure multicouche comprenant des couches alternées de polymères choisis parmi les polyesters, les polymères acryliques, les polyamides, tellles que celles décrites dans les documents EP-A-6921217, EP-A-686858 et US-A-5472798. De telles fibres sont vendues sous les dénominations "Morphotex", « Teijin Tetron Morphotex » par la société TEIJIN.

Les fibres rigides particulièrement préférées sont les fibres de polymide-amide aromatiques.

Des fils ou fibres de polyimide-amide, qui peuvent être utilisés pour les compositions de l'invention, sont décrits, par exemple, dans le document de R. PIGEON et P. ALLARD, Chimie Macromoléculaire Appliquée, 40/41 (1974), pages 139-158 (n° 600), ou bien encore dans les documents US-A-3 802 841, FR-A-2 079 785, EP-A1-0 360 728, EP-A-0 549 494, auquels on pourra se référer.

Les fibres de polyimide-amide aromatique préférées sont des fibres de polyimide-amide comprenant des motifs répétitif de formule : 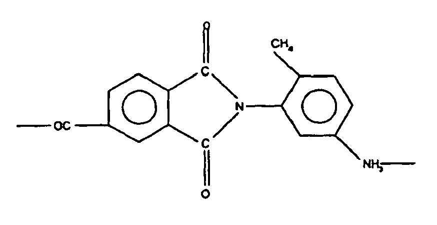 obtenu par polycondensation du toluylène diisocyanate et de l'anhydride triméllitique.

Les fibres peuvent êtres présentes dans la composition selon l'invention en une teneur allant de 0,05 % à 10 % en poids, par rapport au poids total de la composition, de préférence de 0,1% à 5% en poids, et mieux de 0,3 % à 3% en poids.

La composition selon l'invention peut comprendre une cire ou un mélange de cires dites additionnelles, différentes de la cire collante.
Les cires additionnelles susceptibles d'être utilisées dans les compositions selon l'invention sont choisies parmi les cires, solides et rigides à température ambiante, d'origine animale, végétale, minérale ou de synthèse et leurs mélanges.
La cire additionnelle peut également présenter une dureté allant de 0,05 MPa à 30 MPa, et de préférence allant de 6 MPa à 15 MPa, la dureté étant déterminée par la méthode indiquée pour la cire collante.
A titre de cire additionnelle, on peut notamment utiliser les cires hydrocarbonées comme la cire d'abeilles, la cire de lanoline, et les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch et les copolymères cireux ainsi que leurs esters.
On peut aussi citer les cires obtenues par hydrogénation catalytique d'huiles animales ou végétales ayant des chaînes grasses, linéaires ou ramifiées, en C8-C32.
Parmi celles-ci, on peut notamment citer l'huile de jojoba hydrogénée, l'huile de jojoba isomérisée telle que l'huile de jojoba partiellement hydrogénée isomérisée trans fabriquée ou commercialisée par la société Desert Whale sous la référence commerciale ISO-JOJOBA-50^{®}, l'huile de tournesol hydrogénée, l'huile de ricin hydrogénée, l'huile de coprah hydrogénée et l'huile de lanoline hydrogénée, le tétrastéarate de di-(triméthylol-1,1,1 propane) vendu sous la dénomination « HEST 2T-4S » par la société HETERENE, le tétrabéhénate de di-(triméthylol-1,1,1 propane) vendue sous la dénomination HEST 2T-4B par la société HETERENE.
On peut encore citer les cires de silicone, les cires fluorées.
On peut également utiliser la cire obtenue par hydrogénation d'huile d'olive estérifiée avec l'alcool stéarylique vendue sous la dénomination « PHYTOWAX Olive 18 L 57 » ou bien encore les cires obtenues par hydrogénation d'huile de ricin estérifiée avec l'alcool cétylique vendus sous la dénomination « PHYTOWAX ricin 16L64 et 22L73 », par la société SOPHIM. De telles cires sont décrites dans la demande FR-A-2792190.

La cire additionnelle peut être également être présente sous forme de microdispersion de cire telle que décrite plus haut pour la cire collante.

La cire additionnelle peut être présente dans la composition selon l'invention en une teneur allant de 0,1 % à 50 % en poids, par rapport au poids total de la composition, de préférence de 0,5 % à 30 % en poids, et mieux de 1 % à 20 % en poids.

### Milieu cosmétiquement acceptable

Le milieu cosmétiquement acceptable de la composition peut comprendre un solvant volatil, notamment choisi parmi les solvants organiques volatils et les huiles volatiles définis ci-après, et leurs mélanges.

La composition selon l'invention peut comprendre un milieu aqueux, constituant une phase aqueuse, qui peut former la phase continue de la composition.

La phase aqueuse peut être constituée essentiellement d'eau ; elle peut également comprendre un mélange d'eau et de solvant miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, les aldéhydes en C₂-C₄.
La phase aqueuse (eau et éventuellement le solvant organique miscible à l'eau) peut être présente, en une teneur allant de 1 % à 95 % en poids, par rapport au poids total de la composition, de préférence allant de 3 % à 80 % en poids, et préférentiellement allant de 5 % à 60 % en poids.

La composition selon l'invention peut comprendre une huile ou solvant organique qui peut notamment former une phase grasse, et en particulier une phase grasse continue. La composition peut être une composition anhydre.

Par " huile ou solvant organique volatile", on entend au sens de l'invention tout milieu non aqueux susceptible de s'évaporer au contact de la fibre kératinique en moins d'une heure, à température ambiante et pression atmosphérique. Le ou les solvants organiques volatils et les huiles volatiles de invention sont des solvants organiques et des huiles cosmétiques volatiles, liquides à température ambiante, ayant une pression de vapeur non nulle, à température ambiante et pression atmosphérique, allant de 0,13 Pa à 40 000 Pa (10⁻³ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg). Par "huile non volatile", on entend une huile restant sur la fibre kératinique à température ambiante et pression atmosphérique au moins plusieurs heures et ayant notamment une pression de vapeur inférieure à 10⁻³ mm de Hg (0,13Pa).
Ces huiles peuvent être des huiles hydrocarbonées, des huiles siliconées, ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en C₈-C₁₆ le néopentanoate d'iso-hexyle, et leurs mélanges. D'autres huiles hydrocarbonées volatiles comme les distillats de pétrole, notamment ceux vendus sous la dénomination Shell Soit par la société SHELL, peuvent aussi être utilisées. De préférence, le solvant volatil est choisi parmi les huiles volatiles hydrocarbonées ayant de 8 à 16 atomes de carbone et leurs mélanges.

Comme huiles volatiles, on peut aussi utiliser les silicones volatiles, comme par exemple les huiles de silicones linéaires ou cycliques volatiles, notamment celles ayant une viscosité ≤ 6 centistokes (6 10⁻⁶ m²/s), et ayant notamment de 2 à 10 atomes de silicium, ces silicones comportant éventuellement des groupes alkyle ou alkoxy ayant de 1 à 22 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile peut être présente dans la composition selon l'invention en une teneur allant de 0,1 % à 98 % en poids, par rapport au poids total de la composition, de préférence de 1 % à 65 % en poids.

La composition peut également comprendre au moins une huile non volatile, et notamment choisie parmi les huiles hydrocarbonées et/ou siliconées non volatiles.

Comme huile hydrocarbonée non volatile, on peut notamment citer :
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₅ + R₆ soit ≥ 10, comme par exemple l'huile de Purcellin (octanoate de cétostéaryle), le myristate d'isopropyle, le palmitate d'isopropyle, le benzoate d'alcool en C₁₂ à C₁₅, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de diisostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ; et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;

Les huiles non volatiles peuvent être présentes dans la composition selon l'invention en une teneur allant de 0 à 30 % (notamment de 0,1 à 30 %) en poids, de préférence de 0 % à 20 % en poids (notamment 0,1 à 20 %), par rapport au poids total de la composition, et mieux de 0 % à 10 % en poids (notamment 0,1 % à 10 %).

La composition selon l'invention peut comprendre au moins un composé gras pâteux à température ambiante. Par "corps gras pâteux" au sens de l'invention, on entend des corps gras ayant un point de fusion allant de 20 à 55 °C, de préférence 25 à 45°C, et/ou une viscosité à 40 °C allant de 0,1 à 40 Pa.s (1 à 400 poises), de préférence 0,5 à 25 Pa.s, mesurée au Contraves TV ou Rhéomat 80, équipé d'un mobile tournant à 60 Hz. L'homme du métier peut choisir le mobile permettant de mesurer la viscosité, parmi les mobiles MS-r3 et MS-r4, sur la base de ses connaissances générales, de manière à pouvoir réaliser la mesure du composé pâteux testé.

De préférence, ces corps gras sont des composés hydrocarbonés, éventuellement de type polymérique ; ils peuvent également être choisis parmi les composés siliconés; ils peuvent aussi se présenter sous forme d'un mélange de composés hydrocarbonés et/ou siliconés. Dans le cas d'un mélange de différents corps gras pâteux, on utilise de préférence les composés pâteux hydrocarbonés (contenant principalement des atomes de carbone et d'hydrogène et éventuellement des groupements ester), en proportion majoritaire.

Parmi les composés pâteux susceptibles d'être utilisés dans la composition selon l'invention, on peut citer les lanolines et les dérivés de lanoline comme les lanolines acétylées ou les lanolines oxypropylènées ou le lanolate d'isopropyle, ayant une viscosité de 18 à 21 Pa.s, de préférence 19 à 20,5 Pa.s, et/ou un point de fusion de 30 à 55°C et leurs mélanges. On peut également utiliser des esters d'acides ou d'alcools gras, notamment ceux ayant 20 à 65 atomes de carbone (point de fusion de l'ordre de 20 à 35°C et/ou viscosité à 40 °C allant de 0,1 à 40 Pa.s) comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux comme l'acide poly(12-hydroxystéarique) et leurs mélanges.

On peut aussi citer les corps gras pâteux siliconés tels que les polydiméthylsiloxanes (PDMS) ayant des chaînes pendantes du type alkyle ou alcoxy ayant de 8 à 24 atomes de carbone, et un point de fusion de 20-55°C, comme les stearyl dimethicones notamment ceux vendus par la société Dow Corning sous les noms commerciaux de DC2503 et DC25514, et leurs mélanges.

Le corps gras pâteux peut être présent dans la composition selon l'invention en une teneur allant de 0,01 à 60% en poids, par rapport au poids total de la composition, de préférence allant de 0,5 à 45 % en poids, et mieux allant de 2 % à 30 % en poids, dans la composition.

La composition selon l'invention peut contenir des agents tensioactifs émulsionnants présents notamment en une proportion allant de 2 à 30 % en poids par rapport au poids total de la composition, et mieux de 5 % à 15 %. Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition selon l'invention sont choisis :
- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de C₁-C₆ alkyl glucose polyoxyéthylénés, et leurs mélanges.
- parmi les tensioactifs anioniques : les acides gras en C₁₆-C₃₀ neutralisés par les amines, l'ammoniaque ou les sels alcalins, et leurs mélanges.

On utilise de préférence des tensioactifs permettant l'obtention d'émulsion huile-dans-eau ou cire-dans-eau.

La composition selon invention peut comprendre au moins un polymère filmogène.

Le polymère filmogène peut être présent dans la composition selon l'invention en une teneur en matières sèches allant de 0,1 % à 60 % en poids par rapport au poids total de la composition, de préférence de 0,5 % à 40 % en poids, et mieux de 1 % à 30 % en poids.

Dans la présente demande, on entend par "polymère filmogène", un polymère apte à former à lui seul ou en présence d'un agent auxiliaire de filmification, un film continu et adhérent sur un support, notamment sur les matières kératiniques comme les cils.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).
Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₃₀, de préférence en C₁-C₂₀, des (méth)acrylates d'aryle, en particulier d'aryle en C₅-C₁₀, des (méth)acrylates d'hydroxyalkyle, en particulier d'hydroxyalkyle en C₂-C₆.
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C₂-C₁₂. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les poly-uréthanes-polyvinylpirrolidones, les polyester-polyuréthanes, les polyéther-polyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.
L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexanedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement -SO₃M, avec M représentant un atome d'hydrogène, un ion ammonium NH₄⁺ ou un ion métallique, comme par exemple un ion Na⁺, Li⁺, K+, Mg²⁺, Ca²⁺, CU²⁺, Fe²⁺, Fe³⁺. On peut utiliser notamment un monomère aromatique bifonctionnel comportant un tel groupement -SO₃M.

Le noyau aromatique du monomère aromatique bifonctionnel portant en outre un groupement -SO₃M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO₃M : l'acide sulfoisophtalique, l'acide sulfotéréphtalique, l'acide sulfophtalique, l'acide 4-sulfonaphtalène-2,7-dicarboxylique.
On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique.

Les polymères d'origine naturelle, éventuellement modifiés, peuvent être choisis parmi la résine shellac, la gomme de sandaraque, les dammars, les élémis, les copals, les polymères cellulosiques, et leurs mélanges.

Selon un premier mode de réalisation de la composition selon l'invention, le polymère filmogène peut être un polymère hydrosoluble et peut être présent dans une phase aqueuse de la composition ; le polymère est donc solubilisé dans la phase aqueuse de la composition. Comme exemples de polymères filmogènes hydrosolubles, on peut citer :
- les protéines comme les protéines d'origine végétale telles que les protéines de blé, de soja ; les protéines d'origine animale tels que les kératines, par exemples les hydrolysats de kératine et les kératines sulfoniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères d'origine naturelle, éventuellement modifiés, tels que :
   - les gommes arabiques, la gomme de guar, les dérivés du xanthane, la gomme de karaya ;
   - les alginates et les carraghénanes ;
   - les glycoaminoglycanes, l'acide hyaluronique et ses dérivés ;
   - la résine shellac, la gomme de sandaraque, les dammars, les élémis, les copals ;
   - l'acide désoxyribonucléïque ;
   - les muccopolysaccharides tels les chondroïtines sulfate,
et leurs mélanges.

Selon une autre variante de réalisation de la composition selon l'invention, le polymère filmogène peut être un polymère solubilisé dans une phase grasse liquide comprenant des huiles ou solvants organiques tels que ceux décrits précédemment (on dit alors que le polymère filmogène est un polymère liposoluble). Par "phase grasse liquide", on entend, au sens de l'invention, une phase grasse liquide à température ambiante (25°C) et pression atmosphérique (760 mm de Hg, soit 105 Pa), composée d'un ou plusieurs corps gras liquides à température ambiante, appelés aussi huiles, généralement compatibles entre eux.
De préférence, la phase grasse liquide comprend une huile volatile, éventuellement en mélange avec une huile non volatile, les huiles pouvant être choisies parmi les huiles citées précédemment.

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une α-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécane-dioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de la copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, polystéarate de vinyle réticulé à l'aide de divinylbenzène, de diallyléther ou de phtalate de diallyle, poly(méth)acrylate de stéaryle, polylaurate de vinyle, poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en C₂-C₂₀, comme le polybutène, les alkylcelluloses avec un radical alkyle linéaire ou ramifié, saturé ou non en C₁ à C₈ comme l'éthylcellulose et la propylcellulose, les copolymères de la vinylpyrolidone (VP) et notamment les copolymères de la vinylpyrrolidone et d'alcène en C₂ à C₄₀ et mieux en C₃ à C₂₀. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

Le polymère filmogène peut être également présent dans la composition sous la forme de particules en dispersion dans une phase aqueuse ou dans une phase solvant non aqueuse, connue généralement sous le nom de latex ou pseudolatex. Les techniques de préparation de ces dispersions sont bien connues de l'homme du métier.
Comme dispersion aqueuse de polymère filmogène, on peut utiliser les dispersions acryliques vendues sous les dénominations « Neocryl XK-90^{®} », « Neocryl A-1070^{®} », « Neocryl A-1090^{®} », « Neocryl BT-62^{®} », « Neocryl A-1079^{®} » et « Neocryl A-523^{®} » par la société AVECIA-NEORESINS, « Dow Latex 432^{®}» par la société DOW CHEMICAL, « Daitosol 5000 AD^{®} » par la société DAITO KASEY KOGYO; ou encore les dispersions aqueuses de polyuréthane vendues sous les dénominations « Neorez R-981^{®} » et « Neorez R-974^{®} » par la société AVECIA-NEORESINS, les « Avalure UR-405^{®} », « Avalure UR-410^{®} », « Avalure UR-425^{®} », « Avalure UR-450^{®} », « Sancure 875^{®} », « Sancure 861^{®} », « Sancure 878^{®} » et « Sancure 2060^{®} » par la société GOODRICH, « Impranil 85^{®} » par la société BAYER, « Aquamere H-1511^{®} » par la société HYDROMER ; les sulfopolyesters vendus sous le nom de marque « Eastman AQ^{®} » par la société EASTMAN CHEMICAL PRODUCTS, les dispersions vinyliques comme le « Mexomère PAM » et aussi les dispersions acryliques dans l'isododécane comme le « Mexomère PAP » par la société CHIMEX.

La composition selon l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

### Additifs

La composition selon l'invention peut également comprendre une matière colorante comme les matières colorantes pulvérulentes, les colorants liposolubles, les colorants hydrosolubles. Cette matière colorante peut être présente en une teneur allant de 0,01 % à 30 % en poids, par rapport au poids total de la composition.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C orange 5, le jaune quinoléine, le rocou. Les colorants hydrosolubles sont par exemple le jus de betterave, le bleu de méthylène, le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

La composition de l'invention peut comprendre, en outre, tout additif usuellement utilisé en cosmétique tels que les antioxydants, les charges, les conservateurs, les parfums, les neutralisants, les épaississants, les vitamines, et leurs mélanges.

Les charges peuvent être choisies parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de polyamide comme le Nylon^{®} (Orgasol de chez Atochem), de poly-β-alanine et de polyéthylène, les poudres de polymères de tétrafluoroéthylène comme le Téflon^{®}, la lauroyl-lysine, l'amidon, le nitrure de bore, les micro sphères creuses polymériques expansées telles que celles de chlorure de polyvinylidène/acrylonitrile comme l'ExpanceL^{®} (Nobel Industrie), les poudres acryliques telles que le polytrap^{®} (Dow Corning), les particules de polyméthacrylate de méthyle et les microbilles de résine de silicone (Tospearis^{®} de Toshiba, par exemple), le carbonate de calcium précipité, le carbonate et l'hydro-carbonate de magnésium, l'hydroxyapatite, les microsphères de silice creuses (SILICA BEADS^{®} de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.
Les charges peuvent représenter de 0,1 à 25 %, et mieux de 1 à 20 % en poids du poids total de la composition.

De préférence, la composition selon l'invention est un mascara.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

La composition selon l'invention peut être fabriquée par les procédés connus, généralement utilisés dans le domaine cosmétique.

La composition selon l'invention peut être conditionnée dans un produit d'application comprenant un réservoir et un moyen amovible pour fermer, de préférence de manière étanche, ledit réservoir.
Ledit ensemble d'application peut comprendre en outre un organe d'application de la composition de maquillage sur les fibres kératiniques, et notamment les cils, ledit organe d'application permettant le prélèvement de la composition et la restitution de la composition prélevée sur les cils. Cet organe d'application est de préférence solidaire des moyens de fermeture étanche de l'ensemble.
L'ensemble d'application peut également comprendre un organe d'essorage (ou essoreur) dudit organe d'application, l'organe d'essorage pouvant être solidaire du réservoir.

L'organe d'application peut être de préférence une brosse à mascara bien connue de l'homme du métier. Une telle brosse comprend notamment des poils disposés radialement autour d'une âme torsadée, en particulier une âme métallique. La brosse peut être de forme variée et comporter des découpes. Des brosses à mascara sont par exemple décrites dans les documents FR-A-2607373, EP-A-611170, EP-A-811336, EP-A-811337, EP-A-842620.

La figure 1 à laquelle il est maintenant fait référence représente un mode de réalisation préférentiel d'un ensemble de conditionnement et d'application 1 contenant une composition de revêtement des fibres kératiniques selon l'invention.

L'ensemble de conditionnement et d'application 1 comprend un récipient 2 surmonté d'un col fileté 3 dont un bord libre délimite une ouverture 4. Dans l'ouverture 4, est monté un organe d'essorage 5. L'ensemble 1 comprend également un dispositif d'application 10 comprenant un bouchon 11 solidaire d'une tige 13 dont une extrémité comporte un applicateur 12, configuré généralement sous forme d'un arrangement de fibres maintenues entre les deux branches d'un fil de fer torsadé. Une surface intérieure du bouchon 11 est filetée de manière à coopérer avec le filetage du col 3. Ainsi, lorsque l'applicateur 12 et la tige 13 sont disposés à l'intérieur du récipient 2, le filetage du bouchon 11 vient en engagement avec le filetage du col 3 de manière à ce que le bouchon obture de manière étanche l'ouverture 4 du récipient.

Alternativement, l'applicateur peut être constitué d'un peigne comprenant généralement une pluralité de dents obtenues de moulage avec un support en matériau thermoplastique. L'applicateur peut encore être constitué d'un peigne combiné avec une brosse.

L'invention est illustrée plus en détail dans les exemples suivants.

### Exemple 1 :

On a préparé un mascara émulsion cire-dans-eau ayant la composition suivante :
- Cire microcristalline SP18 de la société Strahl and Pitsch 8 g
- Cire d'abeille 1,85 g
- Cire de carnauba 1,2 g
- Cire obtenue par hydrogénation d'huile d'olive estérifiée avec l'alcool stéarylique ( PHYTOWAX Olive 18 L 57) 2 g
- Cire de son de riz 1,6 g
- Triéthanolamine 1,5 g
- Acide stéarique 3 g
- Stéarate de glycéryle 3 g
- Hydroxyéthylcellulose 2 g
- Copolymère PVP/Eicosène 1,5 g
- Polyvinylpyrrolidone 1 g
- Polymethacrylate de sodium (« Darvan 7 » de chez Vanderbilt) 1 g
- mélange de polyuréthane aliphatique, triéthylamine, eau (38/1/61) (AVALURE UR 450 de NOVEON) 13,1 g
- Amidon de maïs modifié en suspension colloïdale dans l'eau à 44% (KM13 Resin de KAMA) 0,95
- Hydroxyethycellulose quaternisée (« Celquat SC 240C » de chez national Starch) 0,1 g
- Chlorure d'alpha gluconamidopropyl diméthyl hydroxyéthyl ammonium en solution aqueuse à 60% (CERAPHYL 60 d'ISP) 0,1 g
- Mélange de polydiméthylsiloxane oxyéthyléné (20 E)-oxypropyléné (20 OP) (Q2-5220 de DOW CORNING) 0,2 g
- Mélange de polydiméthylsiloxane à groupements alpha-omega hydroxylés et cyclopentadiméthylsiloxane 1 g
- Sequioléate de sorbitan 0,25 g
- Polyisobutylène hydrogéné (Parléam SV de NIPPON OIL FATS) 0,75 g
- Butylène glycol 2 g
- Mélange de polydiméthylsiloxane et de silice hydratée (Mirasil SM de RHODIA) 0,2 g
- Fibres de cellulose de 1,3 mm de longueur (Rayon Flock Rcise N0003 MO4 de Claremont Flock Corporation) 0,75 g
- Pigments 6 g
- Ethanol 1 g
- Conservateurs qs
- Eau qsp 100 g

Ce mascara a été jugé comme formant un maquillage lisse et homogène sur les cils, ainsi qu'un effet volumateur et allongeant.

## Revendications

1. Composition de revêtement des fibres kératiniques comprenant, dans un milieu cosmétiquement acceptable, une cire microcristalline ayant un collant supérieur ou égal à 0,7 N.s et une dureté inférieure ou égale à 3,5 MPa, et des fibres.

2. Composition selon la revendication 1, **caractérisée par le fait que** la cire a un collant allant de 0,7 N.s à 30 N.s.

3. Composition selon la revendication 1 ou 2, **caractérisée par le fait que** la cire a un collant supérieur ou égal à 0,8 N.s, de préférence allant de 0,8 N.s à 10 N.s.

4. Composition selon l'une quelconque des revendications précédentes, **caractérisée par le fait que** la cire a un collant allant de 0,8 N.s. à 5 N.s.

5. Composition selon l'une quelconque des revendications précédentes, **caractérisée par le fait que** la cire a une dureté allant de 0,01 à 3,5 MPa.

6. Composition selon l'une quelconque des revendications précédentes, **caractérisée par le fait que** la cire a une dureté allant de 0,05 MPa à 3 MPa, de préférence allant de 0,1 MPa à 2 MPa et mieux de 0, 2 à 1 MPa.

7. Composition selon l'une quelconque des revendications précédentes, **caractérisée par le fait que** la cire microcristalline est présente en une teneur allant de 0,5 % à 60% en poids, par rapport au poids total de la composition, de préférence allant de 2% à 40 % en poids, et préférentiellement allant de 5 % à 20 % en poids.

8. Composition selon l'une des revendications précédentes, **caractérisée en ce que** les fibres sont choisies parmi les fibres de soie, de coton, de laine, de lin, de cellulose, de polyamide, de viscose, d'acétate notamment d'acétate de rayonne, en acrylique notamment de polyméthacrylate de méthyle ou de poly 2-hydroxyéthyl méthacrylate, de polyoléfine et notamment de polyéthylène ou de polypropylène, de silice, de carbone notamment sous forme graphite, de polytétrafluoroéthylène, de collagène insoluble, de polyesters, de polychlorure de vinyle ou de vinylidène, d'alcool polyvinylique, de polyacrylonitrile, de chitosane, de polyuréthane, de polyéthylène phtalate, des fibres de mélanges de polymères, les fibres rigides sensiblement rectilignes les fibres élastomériques et leurs mélanges.

9. Composition selon l'une des revendications précédentes **caractérisée en ce que** les fibres sont des fibres d'origine synthétique.

10. Composition selon l'une des revendications précédentes **caractérisée en ce que** les fibres sont des fibres rigides.

11. Composition selon la revendication précédente, **caractérisée en ce que** les fibres rigides sont des fibres de polyimide-amide aromatique

12. Composition selon la revendication 11 **caractérisée par le fait que** le polyimide-amide est obtenu par polymérisation du tolyulène diisocyanate et de l'anhydride triméllitique et comprend des motifs répétitifs de formule : 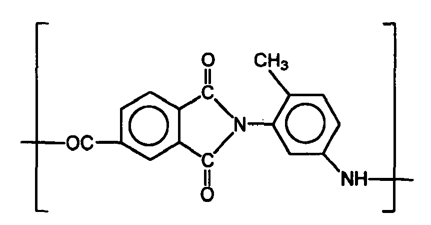 obtenu par polycondensation du toluylène diisocyanate et de l'anhydride triméllitique.

13. Composition selon l'une des revendications précédentes **caractérisée en ce que** les fibres ont une longueur allant de 1 µm à 10 mm, de préférence de 0,1 mm à 5 mm et mieux de 0,3 à 3 mm.

14. Composition selon l'une des revendications précédente, **caractérisée en ce que** les fibres ont un section comprise dans un cercle de diamètre allant de 2 nm à 500 µm, de préférence allant de 100 nm à 100 µm.

15. Composition selon l'une des revendications précédentes, **caractérisée en ce que** les fibres sont présentes en une teneur allant de 0,05 % à 10 % en poids par rapport au poids total de la composition, de préférence de 0,1 à 5%, et mieux de 0,3 à 3% en poids.

16. Composition selon l'une quelconque des revendications précédentes, **caractérisée par le fait qu'**elle comprend une phase aqueuse.

17. Composition selon l'une quelconque des revendications précédentes, **caractérisée par le fait qu'**elle comprend une phase aqueuse formée d'eau ou d'un mélange d'eau et de solvant organique miscible à l'eau.

18. Composition selon la revendication précédente, **caractérisée par le fait que** le solvant organique miscible à l'eau est choisi parmi les monoalcools inférieurs ayant de 1 à 5 atomes de carbone, les glycols ayant de 2 à 8 atomes de carbone, les cétones en C₃-C₄, les aldéhydes en C₂-C₄.

19. Composition selon l'une des revendications 16 à 18, **caractérisée par le fait que** la phase aqueuse est présente en une teneur allant de 1 % à 95 % en poids, par rapport au poids total de la composition, de préférence allant de 3 % à 80 % en poids, et préférentiellement allant de 5 % à 60 % en poids.

20. Composition selon l'une quelconque des revendications précédentes, **caractérisée par le fait qu'**elle comprend une huile volatile.

21. Composition selon la revendication précédente, **caractérisée par le fait que** l'huile volatile est choisies parmi les huiles hydrocarbonées, les huiles siliconées, ou leurs mélanges.

22. Composition selon la revendication 20 ou 21, **caractérisée par le fait que** l'huile volatile est présente en une teneur allant de 0,1 % à 98 % en poids, par rapport au poids total de la composition, de préférence allant de 1 % à 65 % en poids.

23. Composition selon l'une quelconque des revendications précédentes, **caractérisée par le fait qu'**elle comprend une huile non volatile.

24. Composition selon la revendication précédente, **caractérisée par le fait que** l'huile non volatile est présente en une teneur allant de 0,1 % à 30 % en poids, de préférence de 0,1 % à 20 % en poids, par rapport au poids total de la composition, et mieux de 0,1 % à 10 % en poids.

25. Composition selon l'une quelconque des revendications précédentes, **caractérisée par le fait qu'**elle comprend un polymère filmogène.

26. Composition selon la revendication précédente, **caractérisée par le fait que** le polymère filmogène est présent en une teneur en matières sèches allant de 0,1 % à 60 % en poids par rapport au poids total de la composition, de préférence allant de 0,5 % à 40 % en poids, et préférentiellement allant de 1 % à 30 % en poids.

27. Composition selon l'une quelconque des revendications précédentes, **caractérisée par le fait qu'**il comprend une cire additionnelle.

28. Composition selon la revendication précédente, **caractérisée par le fait que** la cire additionnelle est présente dans le composition en une teneur allant de 0,1 % à 50 % en poids, par rapport au poids total de la composition, de préférence de 0,5 % à 30 % en poids, et mieux de 1 % à 20 % en poids.

29. Composition selon l'une quelconque des revendications précédentes, **caractérisée par le fait qu'**elle comprend un tensioactif.

30. Composition selon l'une quelconque des revendications précédentes, **caractérisée par le fait qu'**il comprend un additif choisi parmi les matières colorantes, les antioxydants, les charges, les composé gras pâteux, les conservateurs, les parfums, les neutralisants, les épaississants, les vitamines, les agents plastifiants, et leurs mélanges.

31. Composition selon l'une des revendications précédentes, **caractérise par le fait qu'**elle est un mascara.

32. Procédé cosmétique de maquillage ou de soin non thérapeutique des fibres kératiniques comprenant l'application sur les fibres kératiniques d'un composition selon l'une quelconque des revendications 1 à 31.

33. Utilisation d'un composition selon l'une quelconque des revendications 1 à 31 pour obtenir un maquillage des fibres kératiniques homogène et/ou lisse et/ou un effet allongeant et/ou un effet volumateur des fibres kératiniques maquillées.

34. Utilisation d'une cire microcristalline ayant un collant supérieur ou égal à 0,7 N.s et une dureté inférieure ou égale à 3,5 MPa, associée à des fibres dans une composition de mascara, pour obtenir un maquillage des fibres kératiniques homogène et/ou lisse et un effet allongeant et/ ou volumateur des fibres kératiniques maquillés.

35. Ensemble de conditionnement et d'application d'un produit de revêtement des fibres kératiniques comprenant :
i) un récipient contenant une composition selon l'une quelconque des revendications 1 à 31, et
ii) des moyens, notamment sous forme d'une brosse torsadée ou d'un peigne, pour l'application de la composition sur les fibres.

## Claims

1. Composition for coating keratin fibres, comprising, in a cosmetically acceptable medium, a microcrystalline wax with a tack of greater than or equal to 0.7 N.s and a hardness of less than or equal to 3.5 MPa, and fibres.

2. Composition according to Claim 1, **characterized in that** the wax has a tack ranging from 0.7 N.s to 30 N.s.

3. Composition according to Claim 1 or 2, **characterized in that** the wax has a tack of greater than or equal to 0.8 N.s, preferably ranging from 0.8 N.s to 10 N.s.

4. Composition according to any one of the preceding claims, **characterized in that** the wax has a tack ranging from 0.8 N.s to 5 N.s.

5. Composition according to any one of the preceding claims, **characterized in that** the wax has a hardness ranging from 0.01 to 3.5 MPa.

6. Composition according to any one of the preceding claims, **characterized in that** the wax has a hardness ranging from 0.05 MPa to 3 MPa, preferably ranging from 0.1 MPa to 2 MPa and better still from 0.2 to 1 MPa.

7. Composition according to any one of the preceding claims, **characterized in that** the microcrystalline wax is present in a content ranging from 0.5% to 60% by weight, preferably ranging from 2% to 40% by weight and preferentially ranging from 5% to 20% by weight relative to the total weight of the composition.

8. Composition according to one of the preceding claims, **characterized in that** the fibres are chosen from silk fibre, cotton fibre, wool fibre, flax fibre, cellulose fibre, polyamide fibre viscose fibre, acetate fibre, in particular rayon acetate fibre, acrylic fibre, in particular polymethyl methacrylate fibre or poly(2-hydroxyethyl methacrylate) fibre, polyolefin fibre and in particular polyethylene or polypropylene fibre, silica fibre, carbon fibre, in particular in graphite form, polytetrafluoroethylene fibre, insoluble collagen fibre, polyester fibre, polyvinyl chloride fibre or polyvinylidene chloride fibre, polyvinyl alcohol fibre, polyacrylonitrile fibre, chitosan fibre, polyurethane fibre, polyethylene phthalate fibre, fibres formed from mixtures of polymers, substantially rectilinear rigid fibres and elastomeric fibres, and mixtures thereof.

9. Composition according to one of the preceding claims, **characterized in that** the fibres are fibres of synthetic origin.

10. Composition according to one of the preceding claims, **characterized in that** the fibres are rigid fibres.

11. Composition according to the preceding claim, **characterized in that** the rigid fibres are aromatic polyimide-amide fibres.

12. Composition according to Claim 11, **characterized in that** the polyimide-amide is obtained by polymerization of tolylene diisocyanate and trimellitic anhydride, and comprises repeating units of formula: 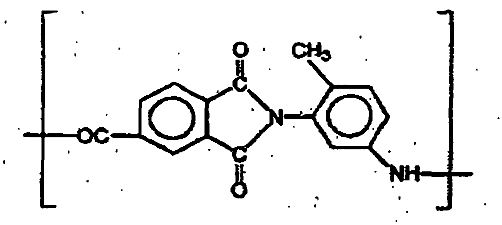 obtained by polycondensation of tolylene diisocyanate and trimellitic anhydride.

13. Composition according to one of the preceding claims, **characterized in that** the fibres have a length ranging from 1 µm to 10 mm, preferably from 0.1 mm to 5 mm and better still from 0.3 to 3 mm.

14. Composition according to one of the preceding claims, **characterized in that** the fibres have a cross section that is within a circle of diameter ranging from 2 nm to 500 µm and preferably ranging from 100 nm to 100 µm.

15. Composition according to one of the preceding claims, **characterized in that** the fibres are present in a content ranging from 0.05% to 10%, preferably from 0.1% to 5% and better still from 0.3% to 3% by weight relative to the total weight of the composition.

16. Composition according to any one of the preceding claims, **characterized in that** it comprises an aqueous phase.

17. Composition according to any one of the preceding claims, **characterized in that** it comprises an aqueous phase formed from water or a mixture of water and of water-miscible organic solvent.

18. Composition according to the preceding claim, **characterized in that** the water-miscible organic solvent is chosen from lower monoalcohols containing from 1 to 5 carbon atoms, glycols containing from 2 to 8 carbon atoms, C₃-C₄ ketones and C₂-C₄ aldehydes.

19. Composition according to one of Claims 16 to 18, **characterized in that** the aqueous phase is present in a content ranging from 1% to 95% by weight, preferably ranging from 3% to 80% by weight and preferentially ranging from 5% to 60% by weight relative to the total weight of the composition.

20. Composition according to any one of the preceding claims, **characterized in that** it comprises a volatile oil.

21. Composition according to the preceding claim, **characterized in that** the volatile oil is chosen from hydrocarbon-based oils and silicone oils, or mixtures thereof.

22. Composition according to Claim 20 or 21, **characterized in that** the volatile oil is present in a content ranging from 0.1% to 98% by weight and preferably ranging from 1% to 65% by weight relative to the total weight of the composition.

23. Composition according to any one of the preceding claims, **characterized in that** it comprises a non-volatile oil.

24. Composition according to the preceding claim, **characterized in that** the non-volatile oil is present in a content ranging from 0.1% to 30% by weight, preferably from 0.1% to 20% by weight and better still from 0.1% to 10% by weight relative to the total weight of the composition.

25. Composition according to any one of the preceding claims, **characterized in that** it comprises a film-forming polymer.

26. Composition according to the preceding claim, **characterized in that** the film-forming polymer is present in a solids content ranging from 0.1% to 60% by weight, preferably ranging from 0.5% to 40% by weight and preferentially ranging from 1% to 30% by weight relative to the total weight of the composition.

27. Composition according to any one of the preceding claims, **characterized in that** it comprises an additional wax.

28. Composition according to the preceding claim, **characterized in that** the additional wax is present in the composition in a content ranging from 0.1% to 50% by weight, preferably from 0.5% to 30% by weight and better still from 1% to 20% by weight relative to the total weight of the composition.

29. Composition according to any one of the preceding claims, **characterized in that** it comprises a surfactant.

30. Composition according to any one of the preceding claims, **characterized in that** it comprises an additive chosen from dyestuffs, antioxidants, fillers, pasty fatty compounds, preserving agents, fragrances, neutralizes, thickeners, vitamins, plasticizers, and mixtures thereof.

31. Composition according to one of the preceding claims, **characterized in that** it is a mascara.

32. Non-therapeutic cosmetic process for making up or caring for keratin fibres, comprising the application to the keratin fibres of a composition according to any one of Claims 1 to 31.

33. Use of a composition according to any one of Claims 1 to 31 to obtain a uniform and/or smooth makeup result on keratin fibres and/or a lengthening effect and/or a volumizing effect on made-up keratin fibres.

34. Use of a microcrystalline wax with a tack of greater than or equal to 0.7 N.s and a hardness of less than or equal to 3.5MPa, combined with fibres in a mascara composition, to obtain a uniform and/or smooth makeup result on the keratin fibres and a lengthening and/or volumizing effect on made-up keratin fibres.

35. Assembly for packaging and applying a product for coating keratin fibres, comprising:
i) a container containing a composition according to any one of Claims 1 to 31, and
ii) means, especially in the form of a twisted brush or a comb, for applying the composition to the fibres.

## Patentansprüche

1. Zusammensetzung zum Beschichten von Keratinfasern, die in einem kosmetisch akzeptablen Medium ein mikrokristallines Wachs mit einer Klebrigkeit von 0,7 N.s oder darüber und eine Härte von 3,5 MPa oder darunter und Fasern enthält.

2. Zusammensetzung nach Anspruch 1, **dadurch gekennzeichnet, dass** das Wachs eine Klebrigkeit von 0,7 bis 30 N.s hat.

3. Zusammensetzung nach Anspruch 1 oder 2, **dadurch gekennzeichnet, dass** das Wachs eine Klebrigkeit von 0,8 N.s oder darüber und vorzugsweise im Bereich von 0,8 bis 10 N.s aufweist.

4. Zusammensetzung nach einem der vorhergehenden Ansprüche, **dadurch gekennzeichnet, dass** das Wachs eine Klebrigkeit von 0,8 bis 5 N.s aufweist.

5. Zusammensetzung nach einem der vorhergehenden Ansprüche, **dadurch gekennzeichnet, dass** das Wachs eine Härte von 0,01 bis 3,5 MPa besitzt.

6. Zusammensetzung nach einem der vorhergehenden Ansprüche, **dadurch gekennzeichnet, dass** das Wachs eine Härte von 0,05 bis 3 MPa, vorzugsweise 0,1 bis 2 MPA und besser 0,2 bis 1 MPa aufweist.

7. Zusammensetzung nach einem der vorhergehenden Ansprüche, **dadurch gekennzeichnet, dass** das mikrokristalline Wachs in einer Menge von 0,5 bis 60 Gew.-%, bezogen auf das Gesamtgewicht der Zusammensetzung, vorzugsweise 2 bis 40 Gew.-% und noch bevorzugter 5 bis 20 Gew.-% vorliegt.

8. Zusammensetzung nach einem der vorhergehenden Ansprüche, **dadurch gekennzeichnet, dass** die Fasern unter Seidenfasern, Baumwollfasern, Wollfasern, Leinenfasern, Cellulosefasern, Polyamidfasern, Viskosefasern, Acetatfasern und insbesondere Fasern aus Rayonacetat, Acrylfasern und insbesondere Fasern aus Polymethylmethacrylat oder Poly(2-hydroxyethylmethacrylat), Polyolefinfasern und besondern Fasern aus Polyethylen oder Polypropylen, Siliciumoxidfasern, Kohlenstofffasern und besonders Fasern in Form von Graphit, Polytetrafluorethylenfasern, Fasern aus unlöslichem Collagen, Polyesterfasern, Fasern aus Polyvinylchlorid oder Polyvinylidenchlorid, Polyvinylalkoholfasern, Polyacrylnitrilfasern, Chitosanfasern, Polyurethanfasern, Polyethylenphthalatfasern, Fasern aus Gemischen dieser Polymere, in etwa geradlinigen starren Fasern, elastomeren Fasern und deren Gemischen ausgewählt sind.

9. Zusammensetzung nach einem der vorhergehenden Ansprüche, **dadurch gekennzeichnet, dass** es sich bei den Fasern um Fasern synthetischer Herkunft handelt.

10. Zusammensetzung nach einem der vorhergehenden Ansprüche, **dadurch gekennzeichnet, dass** die Fasern starre Fasern sind.

11. Zusammensetzung nach dem vorhergehenden Anspruch, **dadurch gekennzeichnet, dass** es sich bei den starren Fasern um Fasern aus einem aromatischen Polyimidamid handelt.

12. Zusammensetzung nach Anspruch 11, **dadurch gekennzeichnet, dass** das Polyimidamid durch Polymerisation von Tolyulendiisocyanat und Trimellitsäureanhydrid erhalten wird und Wiederholungseinheiten der folgenden Formel aufweist: 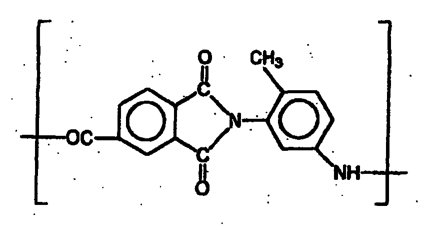 erhalten durch Polykondensation von Toluylendiisocyanat und Trimellitsäureanhydrid.

13. Zusammensetzung nach einem der vorhergehenden Ansprüche, **dadurch gekennzeichnet, dass** die Fasern eine Länge von 1 µm bis 10 mm, vorzugsweise 0,1 bis 5 mm und besser 0,3 bis 3 mm aufweisen.

14. Zusammensetzung nach einem der vorhergehenden Ansprüche, **dadurch gekennzeichnet, dass** die Fasern einen Querschnitt besitzen, der in einen Kreis mit einem Durchmesser von 2 nm bis 500 µm und vorzugsweise 100 nm bis 100 µm einbeschrieben werden kann.

15. Zusammensetzung nach einem der vorhergehenden Ansprüche, **dadurch gekennzeichnet, dass** die Fasern in einer Menge von 0,05 bis 10 Gew.-%, bezogen auf das Gesamtgewicht der Zusammensetzung, vorzugsweise 0,1 bis 5 % und besser 0,3 bis 3 Gew.-% enthalten sind.

16. Zusammensetzung nach einem der vorhergehenden Ansprüche, **dadurch gekennzeichnet, dass** sie eine wässrige Phase enthält.

17. Zusammensetzung nach einem der vorhergehenden Ansprüche, **dadurch gekennzeichnet, dass** sie eine wässrige Phase enthält, die aus Wasser oder einem Gemisch von Wasser und einem mit Wasser mischbaren organischen Lösungsmittel gebildet ist.

18. Zusammensetzung nach dem vorhergehenden Anspruch, **dadurch gekennzeichnet, dass** das mit Wasser mischbare, organische Lösungsmittel unter den niederen Monoalkoholen mit 1 bis 5 Kohlenstoffatomen, Glycolen mit 2 bis 8 Kohlenstoffatomen, Ketonen mit 3 bis 4 Kohlenstoffatomen und Aldehyden mit 2 bis 4 Kohlenstoffatomen ausgewählt ist.

19. Zusammensetzung nach einem der Ansprüche 16 bis 18, **dadurch gekennzeichnet, dass** die wässrige Phase in einer Menge von 1 bis 95 Gew.-%, bezogen auf das Gesamtgewicht der Zusammensetzung, vorzugsweise 3 bis 80 Gew.-% und bevorzugt 5 bis 60 Gew.-% enthalten ist.

20. Zusammensetzung nach einem der vorhergehenden Ansprüche, **dadurch gekennzeichnet, dass** sie ein flüchtiges Öl enthält.

21. Zusammensetzung nach dem vorhergehenden Anspruch, **dadurch gekennzeichnet, dass** das flüchtige Öl unter den Kohlenwasserstoffölen, Siliconölen oder deren Gemischen ausgewählt ist.

22. Zusammensetzung nach Anspruch 20 oder 21, **dadurch gekennzeichnet, dass** das flüchtige Öl in einer Menge von 0,1 bis 98 Gew.-%, bezogen auf das Gesamtgewicht der Zusammensetzung, und vorzugsweise 1 bis 65 Gew.-% enthalten ist.

23. Zusammensetzung nach einem der vorhergehenden Ansprüche, **dadurch gekennzeichnet, dass** sie ein nichtflüchtiges Öl enthält.

24. Zusammensetzung nach dem vorhergehenden Anspruch, **dadurch gekennzeichnet, dass** das nichtflüchtige Öl in einer Menge von 0,1 bis 30 Gew.-%, vorzugsweise 0,1 bis 20 Gew.-%, bezogen auf das Gesamtgewicht der Zusammensetzung, und besser 0,1 bis 10 Gew.-% enthalten ist.

25. Zusammensetzung nach einem der vorhergehenden Ansprüche, **dadurch gekennzeichnet, dass** sie ein filmbildendes Polymer enthält.

26. Zusammensetzung nach dem vorhergehenden Anspruch, **dadurch gekennzeichnet, dass** das filmbildende Polymer in einem Trockensubstanzgehalt von 0,1 bis 60 Gew.-%, bezogen auf das Gesamtgewicht der Zusammensetzung, vorzugsweise 0,5 bis 40 Gew.-% und noch bevorzugter im Bereich von 1 bis 30 Gew.-% enthalten ist.

27. Zusammensetzung nach einem der vorhergehenden Ansprüche, **dadurch gekennzeichnet, dass** sie ein ergänzendes Wachs enthält.

28. Zusammensetzung nach dem vorhergehenden Anspruch, **dadurch gekennzeichnet, dass** das ergänzende Wachs in der Zusammensetzung in einer Menge von 0,1 bis 50 Gew.-%, bezogen auf das Gesamtgewicht der Zusammensetzung, vorzugsweise 0,5 bis 30 Gew.-% und besser 1 bis 20 Gew.-%, enthalten ist.

29. Zusammensetzung nach einem der vorhergehenden Ansprüche, **dadurch gekennzeichnet, dass** sie einen grenzflächenaktiven Stoff enthält.

30. Zusammensetzung nach einem der vorhergehenden Ansprüche, **dadurch gekennzeichnet, dass** sie einen Zusatzstoff enthält, der unter den Farbmitteln, Antioxidantien, Füllstoffen, pastösen Fettsubstanzen, Konservierungsmitteln, Parfums, Neutralisationsmitteln, Verdickungsmitteln, Vitaminen, Weichmachern und deren Gemischen ausgewählt ist.

31. Zusammensetzung nach einem der vorhergehenden Ansprüche, **dadurch gekennzeichnet, dass** es sich um Mascara handelt.

32. Kosmetisches Verfahren zum Schminken oder für die nichttherapeutische Pflege von Keratinfasern, das das Auftragen einer Zusammensetzung nach einem der Ansprüche 1 bis 31 auf die Keratinfasern umfasst.

33. Verwendung einer Zusammensetzung nach einem der Ansprüche 1 bis 31, um an Keratinfasern eine homogene und/oder glatte Schminke und/oder Schminke mit Verlängerungswirkung und/oder volumenvergrößernder Wirkung der geschminkten Keratinfasern zu bilden.

34. Verwendung eines mikrokristallinen Wachses mit einer Klebrigkeit von mindestens 0,7 N.s und einer Härte von höchstens 3,5 MPa in Kombination mit Fasern in einer Mascarazusammensetzung, um an Keratinfasern eine homogene Schminke und/oder glatte Schminke und eine Verlängerungswirkung und/oder volumenvergrößernde Wirkung der geschminkten Keratinfasern zu erzielen.

35. Einheit für die Konfektionierung und Applikation eines Produkts zum Beschichten von Keratinfasern, umfassend:
i) einen Behälter, der eine Zusammensetzung nach einem der Ansprüche 1 bis 31 enthält, und
ii) Mittel, insbesondere in Form einer gedrehten Bürste oder eines Kamms, zum Auftragen der Zusammensetzung auf die Fasern.
